# EUROPEAN PATENT APPLICATION

(11) **EP 4 512 874 A1**
(43) Date of publication of application: **26.02.2025**
(21) Application number: 23791550.9
(22) Date of filing: 06.03.2023
(51) Int. Cl.: C10G 2/00, C07C 1/12, C07C 9/14, C07C 11/02

(54) **FUEL PRODUCTION APPARATUS AND FUEL PRODUCTION METHOD**

(30) Priority: 18.04.2022 JP 2022068407
(71) Applicant: ENEOS Corporation, Chiyoda-ku Tokyo 100-8162 (JP)
(72) Inventor: NAGATAKE, Satoshi, Tokyo 100-8162 (JP); MAYUMI, Kazuya, Tokyo 100-8162 (JP); KISHIDA, Ryo, Tokyo 100-8162 (JP); SHINGU, Masaki, Tokyo 100-8162 (JP); OHUCHI, Tai, Tokyo 100-8162 (JP); MORI, Hirotaka, Tokyo 100-8162 (JP); SHUTO, Fumihiko, Tokyo 100-8162 (JP); TAKAHAMA, Koshi, Tokyo 100-8162 (JP); FUJIMOTO, Keisuke, Tokyo 100-8162 (JP); YOSHIDA, Yoshiyuki, Tokyo 100-8162 (JP)
(74) Representative: Algemeen Octrooi- en Merkenbureau B.V.
(86) International application number: PCT/JP2023/008276
(87) International publication number: WO 2023/203895

(57) **Abstract**

A fuel production apparatus (1) includes: a synthesis gas production unit (2) that produces a synthesis gas containing carbon monoxide and hydrogen by using carbon dioxide and hydrogen; a hydrocarbon production unit (4) that produces a hydrocarbon (C5+) by using the synthesis gas; a first distillation separator (28) that separates at least a first fraction and a second fraction lighter than the first fraction from an effluent from the hydrocarbon production unit (4); a hydrocracking unit (32) that performs a hydrocracking treatment on a portion of the first fraction; and a catalytic cracking unit (34) that performs a catalytic cracking treatment on another portion of the first fraction.

## Description

### TECHNICAL FIELD

The present invention relates to a fuel production apparatus and a fuel production method.

### BACKGROUND ART

A liquid fuel production technique using GTL (Gas to Liquid) is known (see Patent Literature 1). This production technique includes, as an example, a step of producing hydrogen and carbon monoxide from natural gas, and a step of producing a liquid hydrocarbon having a high energy density by Fischer-Tropsch reaction (hereinafter, appropriately referred to as "FT reaction") using, as a feed gas, a synthesis gas containing hydrogen and carbon monoxide.

### RELATED-ART LITERATURE

### NON-PATENT LITERATURE

Patent Literature 1: JP 2008-248179 A

### SUMMARY OF INVENTION

### TECHNICAL PROBLEM

The present inventors have made a series of intensive studies and come up with a new technology for producing various fuels from the products of the FT reaction.

The present invention has been made in view of such a situation, and a purpose thereof is to provide a technology for producing various fuels from products of the FT reaction.

### SOLUTION TO PROBLEM

One embodiment of the present invention relates to a fuel production apparatus. This fuel production apparatus includes: a synthesis gas production unit that produces a synthesis gas containing carbon monoxide and hydrogen by using carbon dioxide and hydrogen; a hydrocarbon production unit that produces a hydrocarbon by using the synthesis gas; a first distillation separator that separates at least a first fraction and a second fraction lighter than the first fraction from an effluent from the hydrocarbon production unit; a hydrocracking unit that performs a hydrocracking treatment on a portion of the first fraction; and a catalytic cracking unit that performs a catalytic cracking treatment on another portion of the first fraction.

Another embodiment of the present invention relates to a fuel production method. This method includes: producing a synthesis gas containing carbon monoxide and hydrogen by using carbon dioxide and hydrogen; producing a hydrocarbon by using the synthesis gas; separating at least a first fraction and a second fraction lighter than the first fraction from an effluent obtained from the producing a hydrocarbon; performing a hydrocracking treatment on a portion of the first fraction; and performing a catalytic cracking treatment on another portion of the first fraction.

Optional combinations of the aforementioned constituting elements, and implementations of the present disclosure in the form of methods, apparatuses, and systems may also be practiced as additional modes of the present disclosure.

### ADVANTAGEOUS EFFECTS OF INVENTION

According to the present invention, various fuels can be produced from products of the FT reaction.

### BRIEF DESCRIPTION OF DRAWINGS

[Fig. 1] Fig. 1 is a schematic diagram of a fuel production apparatus according to an embodiment.
[Fig. 2] Fig. 2 is a schematic diagram of the fuel production apparatus according to the embodiment.
[Figs. 3] Fig. 3A, Fig. 3B, and Fig. 3C are diagrams showing examples of a reaction occurring in a hydrotreating unit. Fig. 3D is a diagram showing an example of a reaction occurring in a hydrocracking unit. Fig. 3E, Fig. 3F, and Fig. 3G are diagrams showing examples of a reaction occurring in a catalytic cracking unit.
[Fig. 4] Fig. 4 is a schematic diagram of a fuel production apparatus according to the first exemplary variation.
[Fig. 5] Fig. 5 is a schematic diagram of a fuel production apparatus according to the second exemplary variation.
[Fig. 6] Fig. 6 is a schematic diagram of a fuel production apparatus according to the third exemplary variation.
[Fig. 7] Fig. 7 is a schematic diagram of a fuel production apparatus according to the fourth exemplary variation.

### DESCRIPTION OF EMBODIMENTS

Hereinafter, the present invention will be described based on preferred embodiments with reference to the drawings. The embodiments do not limit the technical scope of the present invention and are shown for illustrative purposes, and not all the features described in the embodiments and combinations thereof are necessarily essential to the invention. Therefore, regarding the details of the embodiments, many design modifications such as change, addition, deletion, etc., of the constituent elements may be made without departing from the spirit of the invention defined in the claims. New embodiments resulting from added design change will provide the advantages of the embodiments and variations that are combined. In the embodiments, the details for which such design change is possible are emphasized with the notations "according to the embodiment," "in the embodiment," etc. However, design change is also allowed for those without such notations. Optional combinations of the constituting elements described in the embodiments are also valid as embodiments of the present invention. The same or equivalent constituting elements, members, and processes illustrated in each drawing shall be denoted by the same reference numerals, and duplicative explanations will be omitted appropriately. The scales and shapes of parts shown in each figure are set for the sake of convenience in order to facilitate the explanation and shall not be interpreted in a limited manner unless otherwise mentioned. Terms like "first," "second," etc., used in the specification and claims do not indicate an order or importance by any means and are used to distinguish a certain feature from the others. Some of the components in each figure may be omitted if they are not important for explanation.

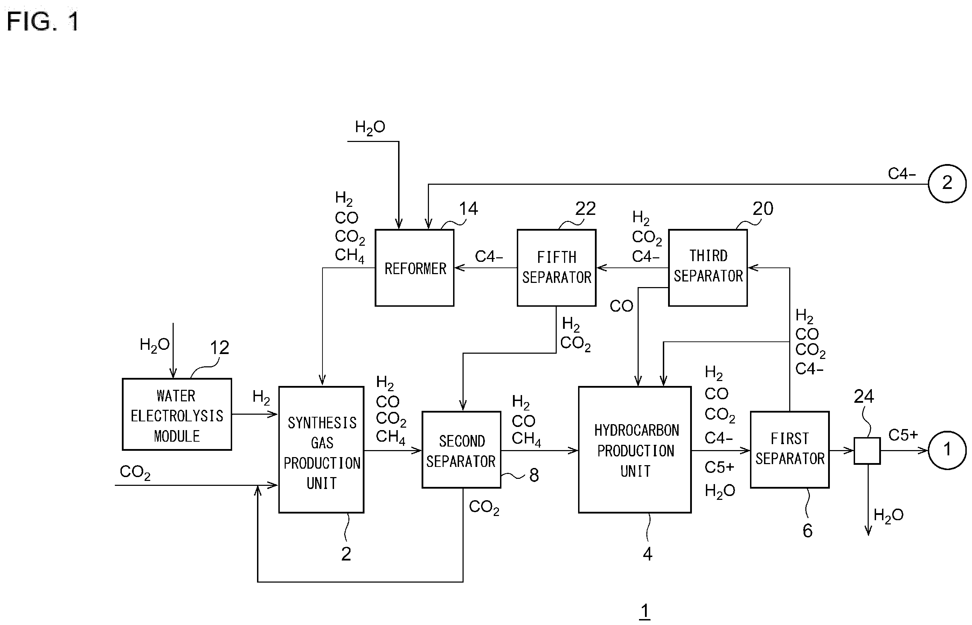

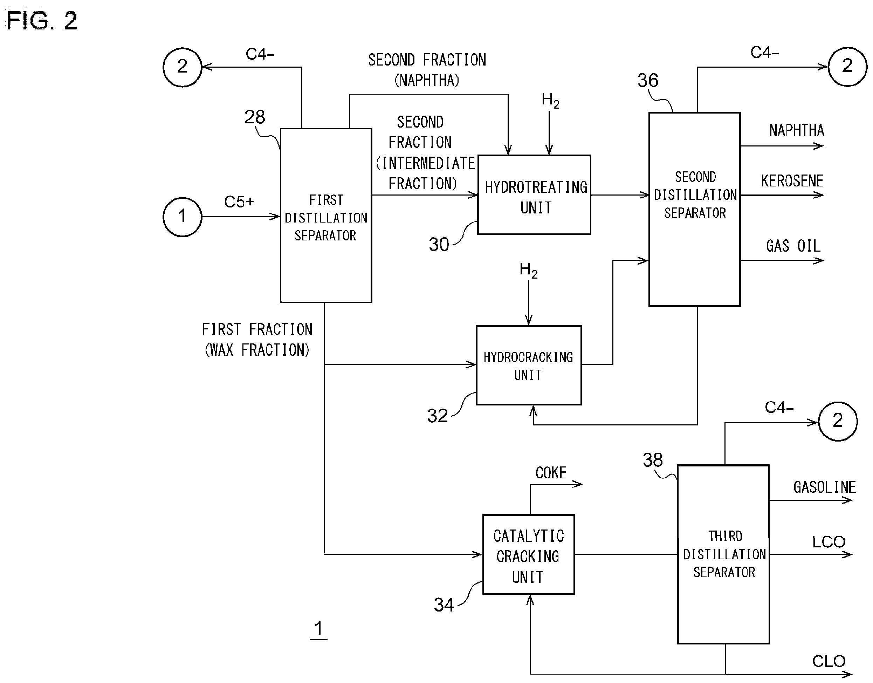

Figs. 1 and 2 are schematic diagrams of a fuel production apparatus 1 according to an embodiment. The fuel production apparatus 1 includes a synthesis gas production unit 2, a hydrocarbon production unit 4, a first separator 6, a second separator 8, a third separator 20, a fifth separator 22, a reformer 14, a first distillation separator 28, a hydrotreating unit 30, a hydrocracking unit 32, a catalytic cracking unit 34, a second distillation separator 36, and a third distillation separator 38.

The synthesis gas production unit 2 is disposed upstream of the hydrocarbon production unit 4. The second separator 8 is disposed between the synthesis gas production unit 2 and the hydrocarbon production unit 4. The first separator 6 is disposed downstream of the hydrocarbon production unit 4. The third separator 20, the fifth separator 22 and the reformer 14 are disposed between the first separator 6 and the synthesis gas production unit 2. Further, the third separator 20 is connected to the hydrocarbon production unit 4, and the fifth separator 22 is connected to the second separator 8.

The first distillation separator 28 is disposed downstream of the first separator 6, sandwiching an oil-water separator 24 therebetween. The hydrotreating unit 30, the hydrocracking unit 32, and the catalytic cracking unit 34 are connected to the first distillation separator 28. The hydrotreating unit 30, the hydrocracking unit 32, and the catalytic cracking unit 34 are connected in parallel with one another to the first distillation separator 28. Further, the hydrotreating unit 30 and the hydrocracking unit 32 are connected to the second distillation separator 36. The catalytic cracking unit 34 is connected to the third distillation separator 38. The physical arrangement of each unit can be set as appropriate. For example, the first separator 6 and the hydrocarbon production unit 4 may be arranged in the same housing. For example, "the second separator 8 is disposed between the synthesis gas production unit 2 and the hydrocarbon production unit 4" does not mean that the second separator 8 is disposed in a space between the synthesis gas production unit 2 and the hydrocarbon production unit 4, but means that the second separator 8 is located in the middle of the flow of materials from the synthesis gas production unit 2 to the hydrocarbon production unit 4. Needless to say, the second separator 8 may be disposed in the space between the synthesis gas production unit 2 and the hydrocarbon production unit 4.

The synthesis gas production unit 2 receives supply of carbon dioxide and hydrogen as feed gases. A synthesis gas containing carbon monoxide and hydrogen is produced by using the carbon dioxide and the hydrogen. As an example, the synthesis gas production unit 2 receives supply of hydrogen from a water electrolysis module 12. The water electrolysis module 12 may be provided as an external device with respect to the fuel production apparatus 1 or may be incorporated in the fuel production apparatus 1.

The water electrolysis module 12 is an electrolyzer that generates hydrogen and oxygen by electrolysis of water. As an example, the water electrolysis module 12 has a structure in which an oxygen generating electrode having a catalyst such as iridium or platinum and a hydrogen generating electrode having a catalyst such as platinum are separated from each other by a membrane having proton conductivity. That is, the water electrolysis module 12 is a solid polymer water electrolysis module. Other examples of the water electrolysis module 12 include an alkaline water electrolysis module and a solid oxide water electrolysis module. The reactions during water electrolysis in the solid polymer water electrolysis module are as shown in the following Formulas (1) and (2).

Reaction taking place at the oxygen generating electrode:

2H₂O → O₂ + 4H⁺ + 4e⁻ (1)

Reaction taking place at the hydrogen generating electrode:

4H⁺ + 4e⁻ → 2H₂ (2)

The water electrolysis module 12 receives supply of electric power necessary for water electrolysis from a power supply device (not illustrated). Examples of the power supply device include a power generation device that generates power using renewable energy, such as a wind power generation device or a solar power generation device. As a result, it is possible to reduce the emission amount of carbon dioxide associated with the production of hydrogen and the production of various types of fuel, which are target products. The power supply device is not limited to a power generation device using renewable energy, and may be a system power supply, a power storage device storing electric power from the renewable energy power generation device or the system power supply, or the like. A combination of two or more of these devices may be used.

As the carbon dioxide supplied to the synthesis gas production unit 2, for example, carbon dioxide recovered from the atmosphere by direct air capture (DAC) can be used. Carbon dioxide separated and recovered from combustion exhaust gas discharged from thermal power generation, a chemical plant, or the like by, for example, a chemical absorption method, a physical absorption method, or the like can be used. As a result, reduction of carbon dioxide in the atmosphere can be expected. The consumption of fossil fuel can be reduced.

In the synthesis gas production unit 2, a reverse shift reaction shown in the following Formula (3) occurs so as to produce a synthesis gas containing at least carbon monoxide and hydrogen. Therefore, the synthesis gas production unit 2 can be considered as a reverse shift reaction unit. The heat required for the reverse shift reaction is supplied, for example, from the outside. As shown in Formula (4) in the following, a part of carbon monoxide may react with hydrogen to generate methane and water. Therefore, methane may also be contained in the synthesis gas, in addition to hydrogen and carbon monoxide. Unreacted carbon dioxide may also be contained in the synthesis gas.

CO₂ + H₂ 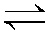 CO + H₂O (3)

CO + 3H₂ 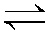 CH₄ + H₂O (4)

The synthesis gas discharged from the synthesis gas production unit 2 is sent to the second separator 8. The water discharged from the synthesis gas production unit 2 may be separated from the synthesis gas and supplied to, for example, the water electrolysis module 12 or the like. The second separator 8 separates carbon dioxide from the synthesis gas. A known carbon dioxide separator can be used for the second separator 8. The carbon dioxide separated by the second separator 8 is supplied to the synthesis gas production unit 2. The synthesis gas production unit 2 receives supply of the carbon dioxide separated by the second separator 8 and also uses the carbon dioxide for production of the synthesis gas. As a result, the utilization rate of carbon dioxide is improved. Therefore, the production efficiency of the fuel can be improved.

The synthesis gas from which carbon dioxide is separated by the second separator 8 is sent to the hydrocarbon production unit 4. The H₂/CO ratio of the synthesis gas supplied to the hydrocarbon production unit 4 is, for example, 1.80 to 2.30, preferably 1.90 to 2.20, and more preferably 2.00 to 2.10. The hydrocarbon production unit 4 produces a hydrocarbon having five or more carbon atoms (hereinafter, referred to as "C5+ component" as appropriate) using the supplied synthesis gas. The C5+ component is, for example, normal paraffin having five or more carbon atoms. Further, the hydrocarbon production unit 4 can also produce olefins, alcohols, and the like.

The hydrocarbon production unit 4 according to the present embodiment is constituted by a known FT reactor. As the FT reactor, a tubular fixed bed reactor, a slurry bed reactor, or the like can be used. In the hydrocarbon production unit 4, the FT reaction shown in the following Formula (5) occurs, and a C5+ component is generated by carbon-carbon chain growth. As a catalyst for FT reaction, a cobalt catalyst, a precipitated iron catalyst, a ruthenium catalyst, or the like can be used. The reaction temperature of the hydrocarbon production unit 4 is, for example, 200°C to 250°C. The proportion of a reaction intermediate having n carbon atoms to be heavy into a reaction intermediate having n + 1 carbon atoms by carbon-carbon chain growth is represented by a chain growth probability α. A higher α means that a higher molecular weight hydrocarbon is obtained. α varies depending on the type of catalyst and reaction conditions, and is preferably 0.75 to 0.95 and more preferably 0.85 to 0.95. In Formula (5), for example, when α is 0.95, n of the C5+ component contained in an amount of 0.1 mol% or more is, for example, an integer of 5 to 60. In the hydrocarbon production unit 4, a gaseous light hydrocarbon having four or less carbon atoms at normal temperature and normal pressure (hereinafter, appropriately referred to as "C4- component") such as methane, ethane, propane, and butane are also produced as by-products.

nCO + (2n + 1)H₂ → CnH₂ₙ₊₂ + nH₂O (5)

An effluent from the hydrocarbon production unit 4 is sent to the first separator 6. The effluent may contain not only a C5+ component and a C4- component, but also water as other by-products, unreacted hydrogen, carbon monoxide, carbon dioxide, and the like. The first separator 6 can be constituted by a known gas-liquid separator, and separates the effluent into a liquid component and a gas component. The liquid component contains a C5+ component and water. The gas component contains hydrogen, carbon monoxide, carbon dioxide, and a C4- component. The gas component may contain a gaseous C5+ component. As for the liquid component, water is separated by a known oil-water separator 24. The liquid component from which water is separated by the oil-water separator 24 is sent to the first distillation separator 28. The separated water may be supplied to, for example, the water electrolysis module 12 or the like. The flow in and after the first distillation separator 28 will be described in detail later.

The gas component is used as a recycle gas. In the present embodiment, a portion of the recycle gas is directly returned from the first separator 6 to the hydrocarbon production unit 4. The hydrocarbon production unit 4 receives supply of the part of the recycle gas from the first separator 6 and also uses the recycle gas for production of the C5+ component. The conversion of carbon monoxide in one reaction in the hydrocarbon production unit 4, that is, when the synthesis gas is passed through the hydrocarbon production unit 4 once is, for example, about 50 to 60%. By returning the part of the recycle gas from the first separator 6 to the hydrocarbon production unit 4, the conversion can be increased to, for example, about 80 to 99%, 85% to 97%, or 90% to 95%. By returning the part of the recycle gas directly from the first separator 6 to the hydrocarbon production unit 4, a load applied to the third separator 20 and the fifth separator 22 can be reduced, and an increase in facility scale of these separators can be suppressed. An increase in facility scale of the synthesis gas production unit 2, the second separator 8, the reformer 14, and the like to which the separated recycle gas is supplied can be suppressed. Therefore, the process efficiency in the fuel production apparatus 1 can be improved.

The remaining recycle gas flowing out of the first separator 6 is sent to the third separator 20 provided between the first separator 6 and the synthesis gas production unit 2. The third separator 20 separates carbon monoxide from the recycle gas. As the third separator 20, for example, a known separator can be used that separates carbon monoxide by a pressure swing adsorption (PSA) method. The carbon monoxide separated by the third separator 20 is supplied to the hydrocarbon production unit 4. The hydrocarbon production unit 4 also uses the carbon monoxide for production of the C5+ component. As a result, the utilization rate of carbon monoxide is improved, and the production efficiency of the C5+ component is improved. By separation of carbon monoxide by the third separator 20, a load applied to the fifth separator 22 can be reduced, and an increase in facility scale of the fifth separator 22 can be suppressed. An increase in facility scale of the synthesis gas production unit 2, the second separator 8, the reformer 14, and the like to which the separated recycle gas is supplied can be suppressed. Therefore, the process efficiency in the fuel production apparatus 1 can be improved.

The recycle gas from which carbon monoxide has been separated by the third separator 20 is sent to the fifth separator 22 provided between the third separator 20 and the synthesis gas production unit 2. The fifth separator 22 separates hydrogen and carbon dioxide from the recycle gas supplied from the third separator 20. As the fifth separator 22, for example, a known separator that separates hydrogen and carbon dioxide by a membrane separation method can be used. As an example, the fifth separator 22 includes at least one of a polyimide film, a carbon film obtained by carbonizing the polyimide film, or a metal film containing Pd. The hydrogen and carbon dioxide separated by the fifth separator 22 are supplied to the second separator 8. As a result, the utilization rate of hydrogen and carbon dioxide is improved, and the production efficiency of the C5+ component is improved. By separation of hydrogen and carbon dioxide by the fifth separator 22, a load applied to the reformer 14 can be reduced, and an increase in facility scale can be suppressed. Therefore, the process efficiency in the fuel production apparatus 1 can be improved.

The second separator 8 separates hydrogen and carbon dioxide supplied from the fifth separator 22 into hydrogen and carbon dioxide respectively. The hydrogen separated by the second separator 8 is supplied to the hydrocarbon production unit 4. The hydrocarbon production unit 4 also uses the hydrogen for production of the C5+ component. As a result, the utilization rate of hydrogen is improved, and the production efficiency of the C5+ component is improved. The carbon dioxide separated by the second separator 8 is supplied to the synthesis gas production unit 2. The synthesis gas production unit 2 also uses the carbon dioxide for production of the synthesis gas. As a result, the utilization rate of carbon dioxide is improved, and the production efficiency of the C5+ component is improved.

The recycle gas from which hydrogen and carbon dioxide have been separated by the fifth separator 22 is sent to the reformer 14 provided between the fifth separator 22 and the synthesis gas production unit 2. As the reformer 14, a known reformer capable of reforming the C4- component into methane can be used. For example, a steam reformer can be used as the reformer 14. Water necessary for the reaction in the reformer 14 is supplied from the outside, for example. Water generated in the synthesis gas production unit 2 or the hydrocarbon production unit 4 may be recycled and supplied to the reformer 14. The reaction temperature of the reformer 14 is, for example, 450°C to 600°C and preferably 450°C to 500°C. By treating the C4- component at 450°C to 600°C, deposition of carbon (coke) can be suppressed. As a result, it is possible to reduce the possibility of occurrence of catalyst deactivation and blockage of the reaction device in the reformer 14.

In the reformer 14, the reactions shown in the above Formulas (6), (7), and (8) occur, and the C4- component (expressed as CₙHₘ in Formula (6)) contained in the recycle gas is reformed into methane, hydrogen, carbon monoxide, and carbon dioxide. In Formula (6), n is an integer of 1 to 4, and m is an integer of 4 to 10. By providing the reformer 14, it is possible to reduce the possibility of occurrence of catalyst deactivation and blockage of the reaction device in the synthesis gas production unit 2.

CₙHₘ + nH₂O → nCO + (n + m/2)H₂ (6)

CO + 3H₂ 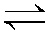 CH₄ + H₂O (7)

CO + H₂O 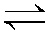 CO₂ + H₂ (8)

The recycle gas containing methane, hydrogen, carbon monoxide, and carbon dioxide generated by the reformer 14 is sent to the synthesis gas production unit 2. The synthesis gas production unit 2 receives supply of the recycle gas and also uses methane, hydrogen, carbon monoxide, and carbon dioxide contained in the recycle gas for production of the synthesis gas. As a result, the utilization rate of the C4- component is improved, and the production efficiency of the C5+ component is improved. Further, when the reaction temperature of the synthesis gas production unit 2 is set to 700°C or higher, preferably 800°C or higher, and more preferably 1000°C or higher, a synthesis gas can be efficiently produced from methane and water by the reverse reaction of the reaction shown in the above Formula (4). As a result, the utilization rate of the C4- component is improved, and the production efficiency of the C5+ component is improved.

The reformer 14 can be supplied with the C4-component separated by the first distillation separator 28, the second distillation separator 36, and/or the third distillation separator 38. The reformer 14 also reforms the C4- component into methane, hydrogen, carbon monoxide, and carbon dioxide. As a result, the utilization rate of the C4-component is improved, and the production efficiency of the C5+ component is improved. The C4- components discharged from each distillation separator may be supplied to the reformer 14 after confluence, or may be supplied to the reformer 14 independently of each other.

Only one of the following may be carried out: the recycle gas separated in the first separator 6 is supplied to the synthesis gas production unit 2 and used to produce a synthesis gas; and the recycled gas is supplied to the hydrocarbon production unit 4 and used to produce hydrocarbons.

Next, the flow in and after the first distillation separator 28 will be described. The first distillation separator 28 is supplied with an effluent from the hydrocarbon production unit 4, more specifically, an effluent from which the recycle gas is separated in the first separator 6. In the present embodiment, the effluent (liquid component containing the C5+ component) from which the recycle gas is separated by the first separator 6, and water is separated in the oil water separator 24 is sent to the first distillation separator 28. The first distillation separator 28 separates at least first and second fractions from the effluent. The second fraction is a lighter fraction than the first fraction. The first distillation separator 28 can be constituted by a known distillation apparatus.

The first fraction according to the present embodiment includes a wax fraction. The second fraction includes a naphtha fraction and an intermediate fraction. The first distillation separator 28 according to the present embodiment also separates the naphtha fraction and the intermediate fraction contained in the second fraction. The naphtha fraction includes, as an example, a liquid hydrocarbon having a boiling point of 150°C or less (for example, five to nine carbon atoms). The intermediate fraction includes, as an example, a hydrocarbon having a boiling point of more than 150°C to 360°C (10 to 21 carbon atoms). The wax fraction includes, as an example, a hydrocarbon having a boiling point of more than 360°C (22 or more carbon atoms). Each fraction may be mixed with hydrocarbons mainly contained in other fractions. Further, the first distillation separator 28 also separates the C4- component that could not be separated by the first separator 6 from the effluent. This C4- component is supplied to the reformer 14 as necessary.

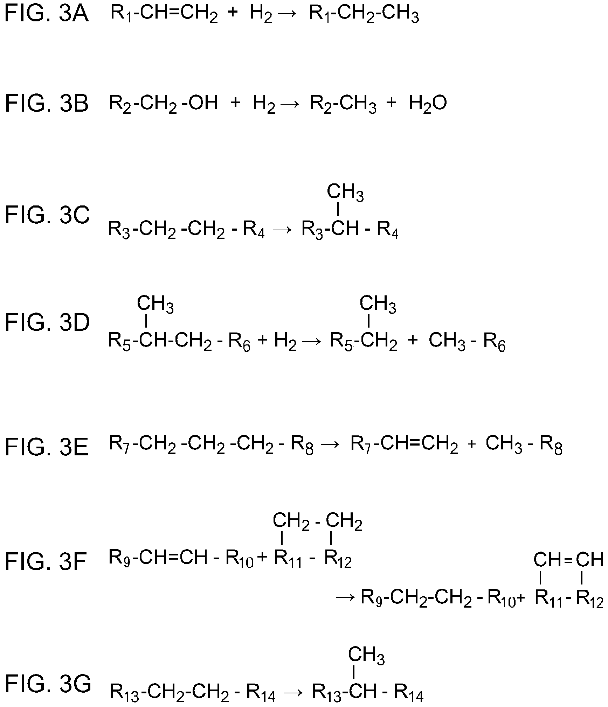

The second fraction separated by the first distillation separator 28 is sent to the hydrotreating unit 30. The hydrotreating unit 30 receives supply of hydrogen from the outside and performs a hydrogenation treatment on the second fraction. The hydrogen may be supplied from the water electrolysis module 12. The hydrotreating unit 30 can be constituted by a known hydrogenation treatment apparatus. Fig. 3A, Fig. 3B, and Fig. 3C are diagrams showing examples of a reaction occurring in the hydrotreating unit 30. Examples of a catalyst used in the hydrotreating unit 30 include a metal having hydrogenation ability, such as iridium, nickel, platinum, or palladium, supported on a carrier such as a metal oxide. As shown in FIG. 3A, the olefin in the second fraction is converted to paraffin by the hydrogenation treatment in the hydrotreating unit 30. Further, as shown in FIG. 3B, the alcohol in the second fraction is converted to paraffin. Thereby, the oxidation stability of the second fraction is improved. Further, as shown in FIG. 3C, n-paraffin in the second fraction is converted to i-paraffin. Thereby, the low-temperature fluidity of the second fraction is improved. The second fraction subjected to the hydrogenation treatment by the hydrotreating unit 30 is sent to the second distillation separator 36.

A portion of the first fraction separated by the first distillation separator 28 is sent to the hydrocracking unit 32. The hydrocracking unit 32 receives supply of hydrogen from the outside and performs a hydrocracking treatment on the first fraction. The hydrogen may be supplied from the water electrolysis module 12. The hydrocracking unit 32 can be constituted by a known hydrogenation treatment apparatus. Fig. 3D is a diagram showing an example of a reaction occurring in the hydrocracking unit 32. Examples of a catalyst used in the hydrocracking unit 32 include a combination of a metal having hydrogenation ability, such as iridium, nickel, platinum, and palladium, and a solid acid catalyst having cracking ability, such as silica-alumina and zeolite. The metal having hydrogenation ability may be supported on a carrier such as a solid acid catalyst having cracking ability (first aspect), or may be supported on a carrier such as a metal oxide having no cracking ability (second aspect). Further, the catalyst used in the hydrocracking unit 32 may be a solid acid catalyst not carrying a metal (third aspect). The first aspect can be used alone. The second and third aspects are each used in combination with other aspects. When two or more catalysts are used, the two or more catalysts may be physically mixed or stacked. As shown in Fig. 3D, long-chain paraffin is cracked by the hydrocracking treatment in the hydrocracking unit 32. Thereby, the lightening of the first fraction is achieved. Further, as in the hydrogenation treatment in the hydrotreating unit 30, olefin and alcohol in the first fraction are converted into paraffin. Further, n-paraffin in the first fraction is converted to i-paraffin. Thereby, the oxidation stability and low-temperature fluidity of the first fraction are improved. The first fraction subjected to the hydrocracking treatment by the hydrocracking unit 32 is sent to the second distillation separator 36.

The second fraction subjected to the hydrogenation treatment by the hydrotreating unit 30 and the first fraction subjected to the hydrocracking treatment by the hydrocracking unit 32 may be sent to separate distillation separators. For example, the second fraction subjected to the hydrogenation treatment by the hydrotreating unit 30 is sent to the second distillation separator 36, and the first fraction subjected to the hydrocracking treatment by the hydrocracking unit 32 may be sent to a fourth distillation separator (not shown) different from the second distillation separator 36 and the third distillation separator 38.

The second distillation separator 36receives supply of the second fraction subjected to the hydrogenation treatment by the hydrotreating unit 30 and the first fraction subjected to the hydrocracking treatment by the hydrocracking unit 32 and distills the second fraction and the first fraction. The second distillation separator 36 can be constituted by a known distillation apparatus. The second distillation separator 36 separates at least one selected from the group consisting of a C4- component, a naphtha fraction, a kerosene fraction (kerosene, jet fuel), a gas oil fraction, or a fraction heavier than a gas oil. In the present embodiment, as an example, all the C4- component, the naphtha fraction, the kerosene fraction, the gas oil fraction, and the fraction heavier than the gas oil are separated. As a result, kerosene or gas oil serving as a fuel is obtained. The C4- component is supplied to the reformer 14 as necessary. A portion or all of the fraction heavier than the gas oil is returned to the hydrocracking unit 32 as necessary. The hydrocracking unit 32 receives supply of the portion or all of the fraction heavier than the gas oil and also performs hydrocracking treatment on the fraction.

The other portion of the first fraction separated by the first distillation separator 28 is sent to the catalytic cracking unit 34. For example, all of the first fraction except for the portion sent to the hydrocracking unit 32 is sent to the catalytic cracking unit 34. The catalytic cracking unit 34 performs a catalytic cracking treatment on the first fraction. The catalytic cracking unit 34 can be constituted by a known catalytic cracking apparatus. As an example, the catalytic cracking unit 34 is constituted by a fluidized bed catalytic cracking apparatus (FCC). However, the catalytic cracking unit 34 is not particularly limited to this configuration and may be of a fixed bed type or the like. Fig. 3E, Fig. 3F, and Fig. 3G are diagrams showing examples of a reaction occurring in the catalytic cracking unit 34. As shown in Fig. 3E, long-chain paraffin is cracked by the catalytic cracking treatment in the catalytic cracking unit 34 so as to generate olefin. Further, as shown in Fig. 3F, hydrogen is transferred between hydrocarbon molecules so as to produce an aromatic compound, for example. Further, the transfer of hydrogen between hydrocarbon molecules, not limited to cyclic hydrocarbons, can produce compounds other than aromatic compounds. Further, as shown in Fig. 3G, the n-paraffin in the first fraction is converted to i-paraffin in the same manner as in the hydrogenation treatment in the hydrotreating unit 30. As a result, the first fraction after the catalytic cracking contains a large amount of olefin and aromatic compounds. The chemical formulas shown in Figs. 3A to 3G are examples and are not limited to the structures listed. Further, R₁ to R₁₄ in Figs. 3A to 3G are hydrocarbon groups that may have substituents on the side chain and may be the same or different from one another.

The first fraction subjected to the catalytic cracking treatment by the catalytic cracking unit 34 is sent to the third distillation separator 38. Further, a coke can be generated by the catalytic cracking treatment. This coke is discharged out of the system from the catalytic cracking unit 34. An example of the method for discharging the coke is a method of supplying oxygen to the catalytic cracking unit 34 so as to burn the coke and discharging it as carbon dioxide or carbon monoxide. The third distillation separator 38 receives supply of the first fraction subjected to the catalytic cracking treatment by the catalytic cracking unit 34 and distills the first fraction. The third distillation separator 38 can be constituted by a known distillation apparatus. The third distillation separator 38 separates at least one selected from the group consisting of a C4- component, a gasoline fraction, a relatively light fraction of heavy oil (LCO: light cycle oil), or a relatively heavy fraction of heavy oil (CLO: clarified oil). In the present embodiment, as an example, all the C4- component, the gasoline fraction, the light cycle oil, and the clarified oil are separated. As a result, gasoline and heavy oil serving as fuel can be obtained. The C4-component is supplied to the reformer 14 as necessary. A portion or all of the CLO is returned to the catalytic cracking unit 34 as necessary. The catalytic cracking unit 34 receives supply of the portion or all of the clarified oil and also performs the catalytic cracking treatment on the clarified oil. LCO can be used, for example, as a heavy oil base material or a gas oil base material. CLO can be used, for example, as a heavy oil base material.

As explained above, the fuel production apparatus 1 according to the present embodiment distributes the first fraction separated by the first distillation separator 28 to the hydrocracking unit 32 and the catalytic cracking unit 34. Then, the kerosene fraction, the gas oil fraction, and the like are separated from the first fraction subjected to the hydrocracking treatment by the hydrocracking unit 32, and the gasoline fraction, the heavy oil fraction, and the like are separated from the first fraction subjected to the catalytic cracking treatment by the catalytic cracking unit 34. As a result, various fuels such as kerosene, gas oil, gasoline, and heavy oil can be efficiently produced from the products of the FT reaction. In particular, by providing the catalytic cracking unit 34, it is possible to produce a larger amount number of olefin and aromatic compounds with a high octane number suitable for gasoline, compared to a case where only the hydrocracking unit 32 is provided. Therefore, the yield of gasoline can be increased. Further, it is possible to easily adjust the yield of various fuels by adjusting the distribution ratio of the first fraction to the hydrocracking unit 32 and the catalytic cracking unit 34.

Further, the fuel production apparatus 1 supplies the second fraction to the hydrotreating unit 30, and separates the kerosene fraction and the gas oil fraction from the second fraction subjected to the hydrogenation treatment by the hydrotreating unit 30. As a result, various fuels can be produced more efficiently.

The following Exemplary Variations can be listed for the above-described embodiment.

### Exemplary Variation 1

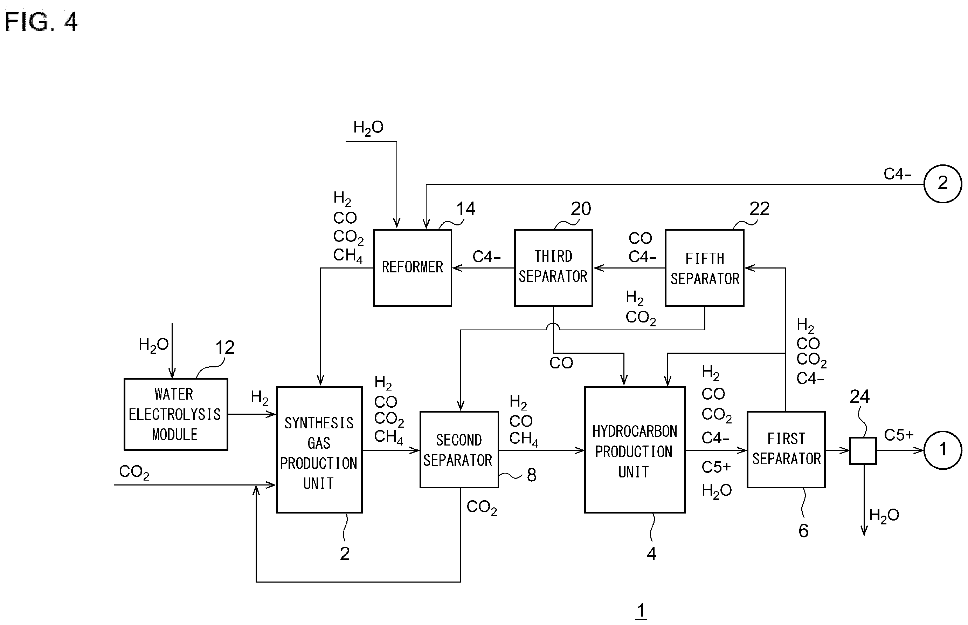

Fig. 4 is a schematic diagram of a fuel production apparatus 1 according to Exemplary Variation 1. The present exemplary variation has a configuration common to the embodiment except that the arrangement of the third separator 20 and the fifth separator 22 is reversed. Hereinafter, the present exemplary variation will be described focusing on a configuration different from that of the embodiment, and description of common configurations will be omitted as appropriate. Moreover, the illustration of the configuration downstream of the first distillation separator 28 is omitted because the configuration is the same as that in the embodiment.

A portion of the recycle gas flowing out of the first separator 6 is directly returned from the first separator 6 to the hydrocarbon production unit 4. The hydrocarbon production unit 4 receives supply of the part of the recycle gas from the first separator 6 and also uses the recycle gas for production of the C5+ component. The remaining recycle gas is sent to the fifth separator 22 provided between the first separator 6 and the synthesis gas production unit 2. The fifth separator 22 separates hydrogen and carbon dioxide from the recycle gas. The hydrogen and carbon dioxide separated by the fifth separator 22 are supplied to the second separator 8. In the fifth separator 22, a part of carbon monoxide in the recycle gas can also be separated.

The second separator 8 separates hydrogen and carbon dioxide supplied from the fifth separator 22 into hydrogen and carbon dioxide respectively. The hydrogen separated by the second separator 8 is supplied to the hydrocarbon production unit 4. The hydrocarbon production unit 4 also uses the hydrogen for production of the C5+ component. The carbon dioxide separated by the second separator 8 is supplied to the synthesis gas production unit 2. The synthesis gas production unit 2 also uses the carbon dioxide for production of the synthesis gas.

The recycle gas from which hydrogen and carbon dioxide have been separated by the fifth separator 22 is sent to the third separator 20 provided between the fifth separator 22 and the synthesis gas production unit 2. The third separator 20 separates carbon monoxide from the recycle gas supplied from the fifth separator 22. The carbon monoxide separated by the third separator 20 is supplied to the hydrocarbon production unit 4. The hydrocarbon production unit 4 also uses the carbon monoxide for production of the C5+ component.

The recycle gas from which carbon monoxide has been separated by the third separator 20 is sent to the reformer 14 provided between the third separator 20 and the synthesis gas production unit 2. The reformer 14 reforms the C4- component contained in the recycle gas into methane, hydrogen, carbon monoxide, and carbon dioxide. The C4- component can be supplied from each distillation separator to the reformer 14. The reformer 14 also reforms the C4- component into methane, hydrogen, carbon monoxide, and carbon dioxide. The recycle gas containing methane, hydrogen, carbon monoxide, and carbon dioxide generated by the reformer 14 is sent to the synthesis gas production unit 2. The synthesis gas production unit 2 also uses methane, hydrogen, carbon monoxide, and carbon dioxide contained in the recycle gas for production of the synthesis gas. When the reaction temperature of the synthesis gas production unit 2 is set to 700°C or higher, preferably 800°C or higher, and more preferably 1000°C or higher, a synthesis gas can be efficiently produced from methane and water by the reverse reaction of the reaction shown in the above Formula (4).

### Exemplary Variation 2

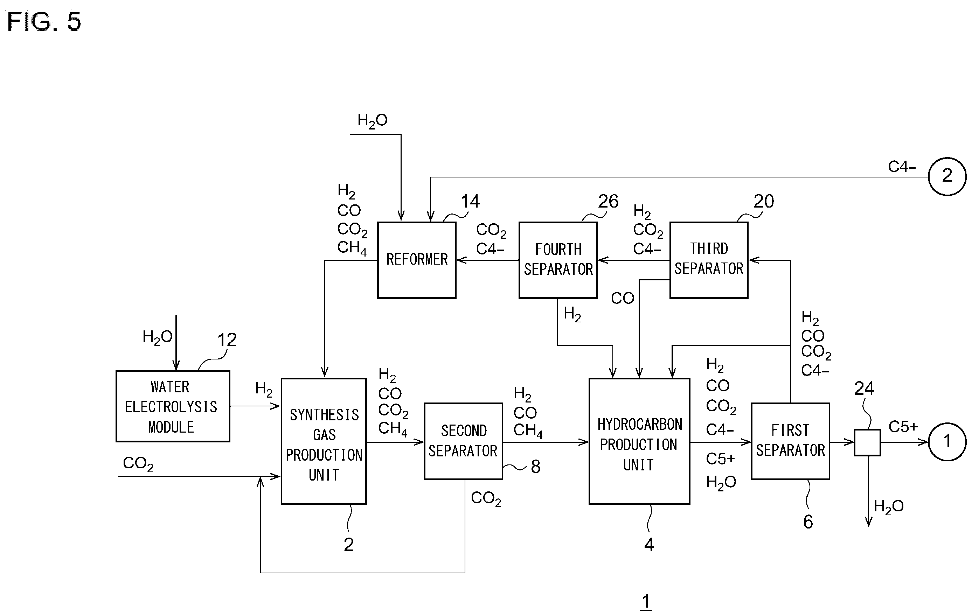

Fig. 5 is a schematic diagram of a fuel production apparatus 1 according to Exemplary Variation 2. The present exemplary variation has a configuration common to the embodiment except that the fuel production apparatus 1 includes a fourth separator 26 instead of the fifth separator 22. Hereinafter, the present exemplary variation will be described focusing on a configuration different from that of the embodiment, and description of common configurations will be omitted as appropriate. Moreover, the illustration of the configuration downstream of the first distillation separator 28 is omitted because the configuration is the same as that in the embodiment.

A portion of the recycle gas flowing out of the first separator 6 is directly returned from the first separator 6 to the hydrocarbon production unit 4. The hydrocarbon production unit 4 receives supply of the part of the recycle gas from the first separator 6 and also uses the recycle gas for production of the C5+ component. The remaining recycle gas is sent to the third separator 20 provided between the first separator 6 and the synthesis gas production unit 2. The third separator 20 separates carbon monoxide from the recycle gas. The carbon monoxide separated by the third separator 20 is supplied to the hydrocarbon production unit 4. The hydrocarbon production unit 4 also uses the carbon monoxide for production of the C5+ component.

The recycle gas from which carbon monoxide has been separated by the third separator 20 is sent to the fourth separator 26 provided between the third separator 20 and the synthesis gas production unit 2. The fourth separator 26 separates hydrogen from the recycle gas supplied from the third separator 20. For the fourth separator 26, a known separator can be used that is for separating hydrogen by, for example, a PSA method or a membrane separation method. The hydrogen separated by the fourth separator 26 is supplied to the hydrocarbon production unit 4. The hydrocarbon production unit 4 also uses the hydrogen for production of the C5+ component. As a result, the utilization rate of hydrogen is improved. By separation of hydrogen by the fourth separator 26, a load applied to the reformer 14 can be reduced, and an increase in facility scale can be suppressed. Therefore, the process efficiency in the fuel production apparatus 1 can be improved.

The recycle gas from which hydrogen is separated by the fourth separator 26 is sent to the reformer 14 provided between the fourth separator 26 and the synthesis gas production unit 2. The reformer 14 reforms the C4- component contained in the recycle gas into methane, hydrogen, carbon monoxide, and carbon dioxide. The C4- component can be supplied from each distillation separator to the reformer 14. The reformer 14 also reforms the C4- component into methane, hydrogen, carbon monoxide, and carbon dioxide. The recycle gas containing methane, hydrogen, carbon monoxide, and carbon dioxide generated by the reformer 14 is sent to the synthesis gas production unit 2. The synthesis gas production unit 2 also uses methane, hydrogen, carbon monoxide, and carbon dioxide contained in the recycle gas for production of the synthesis gas. When the reaction temperature of the synthesis gas production unit 2 is set to 700°C or higher, preferably 800°C or higher, and more preferably 1000°C or higher, a synthesis gas can be efficiently produced from methane and water by the reverse reaction of the reaction shown in the above Formula (4).

### Exemplary Variation 3

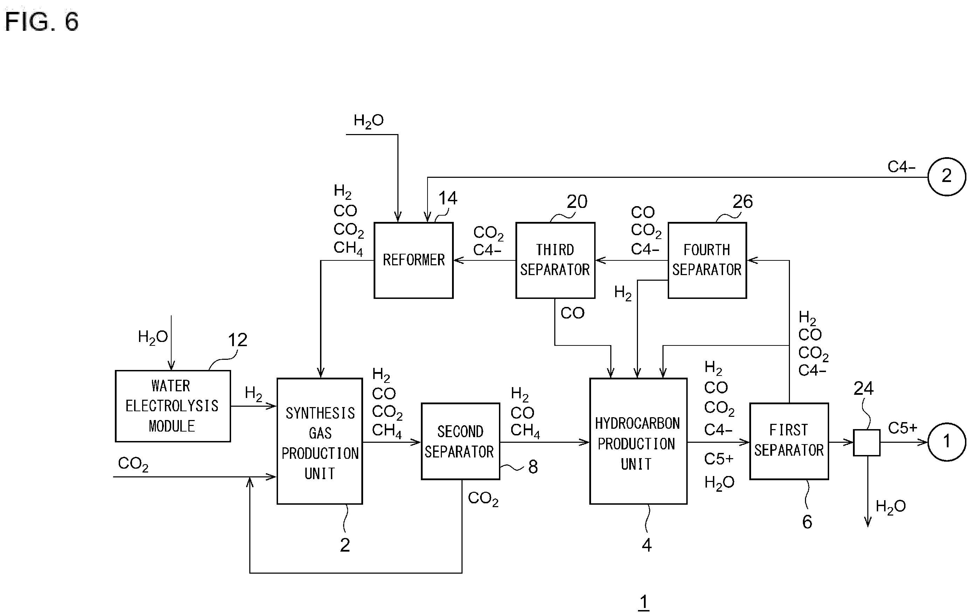

Fig. 6 is a schematic diagram of a fuel production apparatus 1 according to Exemplary Variation 3. The exemplary variation has a configuration common to Exemplary Variation 2 except that the arrangement of the third separator 20 and the fourth separator 26 is reversed. Hereinafter, the present exemplary variation will be described focusing on a configuration different from those of the embodiment and Exemplary Variation 2, and description of common configurations will be omitted as appropriate. Moreover, the illustration of the configuration downstream of the first distillation separator 28 is omitted because the configuration is the same as that in the embodiment.

A portion of the recycle gas flowing out of the first separator 6 is directly returned from the first separator 6 to the hydrocarbon production unit 4. The hydrocarbon production unit 4 receives supply of the part of the recycle gas from the first separator 6 and also uses the recycle gas for production of the C5+ component. The remaining recycle gas is sent to the fourth separator 26 provided between the first separator 6 and the synthesis gas production unit 2. The fourth separator 26 separates hydrogen from the recycle gas. The hydrogen separated by the fourth separator 26 is supplied to the hydrocarbon production unit 4. The hydrocarbon production unit 4 also uses the hydrogen for production of the C5+ component.

The recycle gas from which hydrogen is separated by the fourth separator 26 is sent to the third separator 20 provided between the fourth separator 26 and the synthesis gas production unit 2. The third separator 20 separates carbon monoxide from the recycle gas supplied from the fourth separator 26. The carbon monoxide separated by the third separator 20 is supplied to the hydrocarbon production unit 4. The hydrocarbon production unit 4 also uses the carbon monoxide for production of the C5+ component.

The recycle gas from which carbon monoxide has been separated by the third separator 20 is sent to the reformer 14 provided between the third separator 20 and the synthesis gas production unit 2. The reformer 14 reforms the C4- component contained in the recycle gas into methane, hydrogen, carbon monoxide, and carbon dioxide. The C4- component can be supplied from each distillation separator to the reformer 14. The reformer 14 also reforms the C4- component into methane, hydrogen, carbon monoxide, and carbon dioxide. The recycle gas containing methane, hydrogen, carbon monoxide, and carbon dioxide generated by the reformer 14 is sent to the synthesis gas production unit 2. The synthesis gas production unit 2 also uses methane, hydrogen, carbon monoxide, and carbon dioxide contained in the recycle gas for production of the synthesis gas. When the reaction temperature of the synthesis gas production unit 2 is set to 700°C or higher, preferably 800°C or higher, and more preferably 1000°C or higher, a synthesis gas can be efficiently produced from methane and water by the reverse reaction of the reaction shown in the above Formula (4).

### Exemplary Variation 4

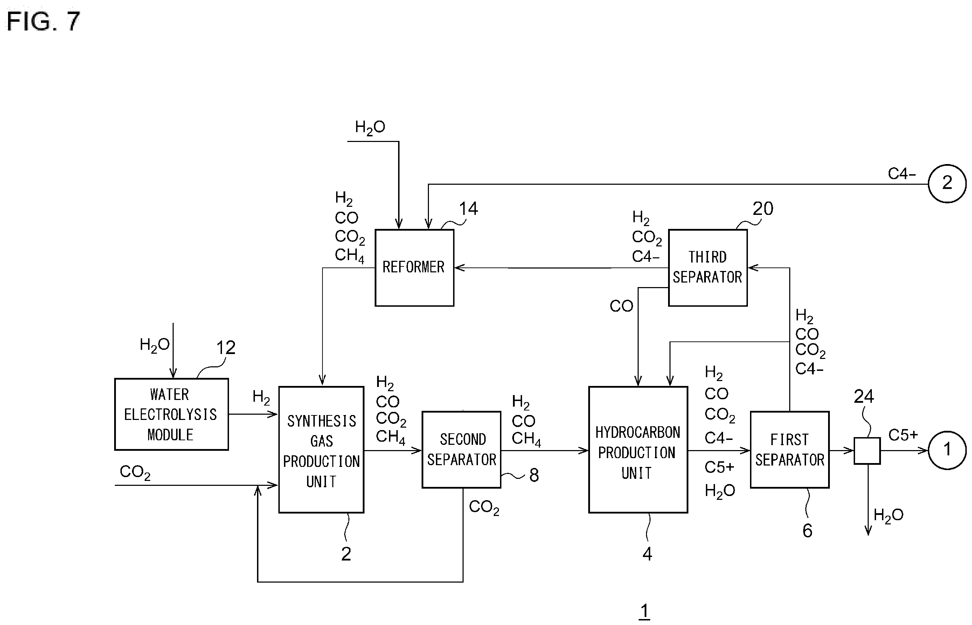

Fig. 7 is a schematic diagram of a fuel production apparatus 1 according to Exemplary Variation 4. The present exemplary variation has a configuration common to the embodiment except that only the third separator 20 is disposed in a recycle gas line from the first separator 6 to the reformer 14. Hereinafter, the present exemplary variation will be described focusing on a configuration different from that of the embodiment, and description of common configurations will be omitted as appropriate. Moreover, the illustration of the configuration downstream of the first distillation separator 28 is omitted because the configuration is the same as that in the embodiment. In the present exemplary variation, the third separator 20 separates carbon monoxide from the recycle gas sent from the first separator 6. The carbon monoxide separated by the third separator 20 is used to produce a C5+ component in the hydrocarbon production unit 4. The recycle gas containing hydrogen, carbon dioxide, and a C4- component from which carbon monoxide has been separated in the third separator 20 is sent to the reformer 14. The reformer 14 reforms the C4- component contained in the recycle gas supplied from the third separator 20 to methane or the like. The recycle gas containing methane or the like generated by the reformer 14 is used to produce synthesis gas in the synthesis gas production unit 2.

### Another Exemplary Variation

A portion of the recycle gas may be discharged outside the system. Hydrogen gas and carbon dioxide gas serving as feed gases supplied to the synthesis gas production unit 2 may contain impurities such as nitrogen and sulfur compounds derived from the respective production sources. Furthermore, reactions in each unit, including the synthesis gas production unit 2, the hydrocarbon production unit 4, and the reformer 14, may result in by-products such as nitrogen compounds and oxygenated compounds. When the recycle gas is repeatedly supplied to the synthesis gas production unit 2, the hydrocarbon production unit 4, and the reformer 14, these impurities and by-products become concentrated, and the catalysts in the units become easily poisoned.

Therefore, the fuel production apparatus 1 is preferably able to intermittently discharge all of the gas out of the system as necessary. Alternatively, the fuel production apparatus 1 is preferably able to constantly or intermittently discharge a portion of the gas out of the system as necessary. The location from which the gas is discharged in the fuel production apparatus 1 can be appropriately set. In the embodiment, shown as examples are: a pipe (for recycling gas) connecting the first separator 6 and the hydrocarbon production unit 4; a pipe connecting the first separator 6 and the third separator 20; a pipe connecting the third separator 20 and the hydrocarbon production unit 4; a pipe connecting the third separator 20 and the fifth separator 22; a pipe connecting the fifth separator 22 and the second separator 8; a pipe connecting the fifth separator 22 and the reformer 14; a pipe connecting the reformer 14 and the synthesis gas production unit 2; a pipe (for recycle CO₂) connecting the second separator 8 and the synthesis gas production unit 2; a pipe (for synthesis gas) connecting the synthesis gas production unit 2 and the second separator 8; a pipe connecting the second separator 8 and the hydrocarbon production unit 4; a pipe connecting the first distillation separator 28 and the reformer 14; a pipe connecting the second distillation separator 36 and the reformer 14; and a pipe connecting the third distillation separator 38 and the reformer 14. The same applies to each exemplary variation, and a pipe connecting separators, a pipe connecting a separator to the hydrocarbon production unit 4, the reformer 14, or the synthesis gas production unit 2, and a pipe connecting a distillation separator and the reformer 14 are shown as examples. In addition, in order to prevent catalyst deactivation, a method of removing impurities and by-products by adsorption or chemical reaction may be also considered.

The present invention may be defined by the items described in the following.

### [Item 1]

A fuel production apparatus (1) comprising:
a synthesis gas production unit (2) that produces a synthesis gas containing carbon monoxide and hydrogen by using carbon dioxide and hydrogen;
a hydrocarbon production unit (4) that produces a hydrocarbon (C5+) by using the synthesis gas; and
a first distillation separator (28) that separates at least a first fraction and a second fraction lighter than the first fraction from an effluent from the hydrocarbon production unit (4);
a hydrocracking unit (32) that performs a hydrocracking treatment on a portion of the first fraction; and
a catalytic cracking unit (34) that performs a catalytic cracking treatment on another portion of the first fraction.

### [Item 2]

The fuel production apparatus (1) according to Item 1, comprising:
a hydrotreating unit (30) that performs a hydrogenation treatment on the second fraction.

### [Item 3]

The fuel production apparatus (1) according to Item 1 or 2, comprising:
a first separator (6) that separates a recycle gas containing a light hydrocarbon (C4-) having four or less carbon atoms from an effluent from the hydrocarbon production unit (4), and
performing at least one of supplying the recycle gas to the synthesis gas production unit (2) so as to also use the recycle gas for producing a synthesis gas or supplying the recycle gas to the hydrocarbon production unit (4) so as to also use the recycle gas for producing a hydrocarbon (C5+).

### [Item 4]

The fuel production apparatus (1) according to Item 3,
wherein the synthesis gas also contains carbon dioxide,
wherein the fuel production apparatus (1) includes a second separator (8) that separates carbon dioxide from the synthesis gas, and
wherein the synthesis gas production unit (2) receives supply of the carbon dioxide separated by the second separator (8) and also uses the carbon dioxide for producing the synthesis gas.

### [Item 5]

The fuel production apparatus (1) according to Item 3 or 4,
wherein the recycle gas also contains carbon monoxide,
wherein the fuel production apparatus (1) includes a third separator (20) that separates carbon monoxide from the recycle gas, and
wherein the hydrocarbon production unit (4) receives supply of the carbon monoxide separated by the third separator (20) and also uses the carbon monoxide for producing the hydrocarbon (C5+).

### [Item 6]

The fuel production apparatus (1) according to any one of Items 3 through 5,
wherein the recycle gas also contains hydrogen,
wherein the fuel production apparatus (1) includes a fourth separator (26) that separates hydrogen from the recycle gas, and
wherein the hydrocarbon production unit (4) receives supply of the hydrogen separated by the fourth separator (26) and also uses the hydrogen for producing the hydrocarbon (C5+).

### [Item 7]

The fuel production apparatus (1) according to Item 4,
wherein the recycle gas also contains hydrogen, carbon monoxide, and carbon dioxide,
wherein the fuel production apparatus (1) includes:
   a third separator (20) that separates carbon monoxide from the recycle gas; and
   a fifth separator (22) that receives supply of the recycle gas from which carbon monoxide has been separated by the third separator (20) and separates hydrogen and carbon dioxide from the recycle gas,
   wherein the second separator (8) receives supply of the hydrogen and carbon dioxide separated by the fifth separator (22) and separates the hydrogen and the carbon dioxide into hydrogen and carbon dioxide respectively, and
   wherein the hydrocarbon production unit (4) receives supply of the carbon monoxide separated by the third separator (20) and the hydrogen separated by the second separator (8) and also uses the carbon monoxide and the hydrogen for production of the hydrocarbon (C5+).

### [Item 8]

The fuel production apparatus (1) according to any one of Items 3 through 6,
wherein the recycle gas also contains hydrogen and carbon monoxide,
wherein the fuel production apparatus (1) includes:
   a third separator (20) that separates carbon monoxide from the recycle gas, and
   a fourth separator (26) that receives supply of the recycle gas from which carbon monoxide has been separated by the third separator (20) and separates hydrogen from the recycle gas, and
   wherein the hydrocarbon production unit (4) receives supply of the carbon monoxide separated by the third separator (20) and the hydrogen separated by the fourth separator (26) and also uses the carbon monoxide and the hydrogen for production of the hydrocarbon (C5+).

### [Item 9]

The fuel production apparatus (1) according to Item 4,
wherein the recycle gas also contains hydrogen, carbon monoxide, and carbon dioxide,
wherein the fuel production apparatus (1) includes:
   a fifth separator (22) that separates hydrogen and carbon dioxide from the recycle gas; and
   a third separator (3) that receives supply of the recycle gas from which hydrogen and carbon dioxide have been separated by the fifth separator (22) and separates carbon monoxide from the recycle gas,
   wherein the second separator (8) receives supply of the hydrogen and carbon dioxide separated by the fifth separator (22) and separates the hydrogen and the carbon dioxide into hydrogen and carbon dioxide respctively, and
   wherein the hydrocarbon production unit (4) receives supply of the carbon monoxide separated by the third separator (20) and the hydrogen separated by the second separator (8) and also uses the carbon monoxide and the hydrogen for production of the hydrocarbon (C5+).

### [Item 10]

The fuel production apparatus (1) according to any one of Items 3 through 6,
wherein the recycle gas also contains hydrogen and carbon monoxide,
wherein the fuel production apparatus (1) includes:
   a fourth separator (26) that separates hydrogen from the recycle gas; and
   a third separator (20) that receives supply of the recycle gas from which hydrogen has been separated by the fourth separator (26) and separates carbon monoxide from the recycle gas, and
   wherein the hydrocarbon production unit (4) receives supply of the hydrogen separated by the fourth separator (26) and the carbon monoxide separated by the third separator (20) and also uses the hydrogen and the carbon monoxide for production of the hydrocarbon (C5+).

### [Item 11]

The fuel production apparatus (1) according to any one of Items 3 through 10, including:
a reformer (14) that reforms the light hydrocarbon (C4-) into methane,
wherein the synthesis gas production unit (2) receives supply of the methane generated by the reformer (14) and also uses the methane for production of the synthesis gas.

### [Item 12]

The fuel production apparatus (1) according to Item 11,
wherein the first distillation separator (28) receives supply of an effluent from which the recycle gas has been separated by the first separator (6) and separates the light hydrocarbon (C4-) from the effluent, and
wherein the reformer (14) receives supply of the light hydrocarbon (C4-) from the first distillation separator (28) and also reforms the light hydrocarbon (C4-) into methane.

### [Item 13]

The fuel production apparatus (1) according to Item 11, comprising:
a hydrotreating unit (30) that performs a hydrogenation treatment on the second fraction;
a second distillation separator (36) that receives supply of the second fraction subjected to the hydrogenation treatment by the hydrotreating unit (30) and the first fraction subjected to the hydrocracking treatment by the hydrocracking unit (32) and distills the second fraction and the first fraction; and
a third distillation separator (38) that receives supply of the first fraction subjected to the catalytic cracking treatment by the catalytic cracking unit (34) and distills the first fraction,
wherein the second distillation separator (36) separates a light hydrocarbon (C4-) from the second fraction and the first fraction,
wherein the third distillation separator (38) separates a light hydrocarbon (C4-) from the first fraction,
wherein the reformer (14) receives supply of a light hydrocarbon (C4-) from at least one of the second distillation separator (36) or the third distillation separator (38) and also reforms the light hydrocarbon (C4-) into methane.

### [Item 14]

The fuel production apparatus (1) according to any one of Items 3 through 13,
wherein the recycle gas also contains carbon monoxide,
wherein the fuel production apparatus (1) includes: a third separator (20) that separates carbon monoxide from the recycle gas, and
a reformer (14) that receives supply of the recycle gas from which carbon monoxide has been separated by the third separator (30) and reforms the light hydrocarbon (C4-) contained in the recycle gas into methane,
wherein the hydrocarbon production unit (4) receives supply of the carbon monoxide separated by the third separator (20) and also uses the carbon monoxide for producing the hydrocarbon (C5+), and
wherein the synthesis gas production unit (2) receives supply of the methane generated by the reformer (14) and also uses the methane for producing the synthesis gas.

### [Item 15]

The fuel production apparatus (1) according to any one of Items 2 through 14, comprising:
a second distillation separator (36) that receives supply of the second fraction subjected to the hydrogenation treatment by the hydrotreating unit (30) and the first fraction subjected to the hydrocracking treatment by the hydrocracking unit (32) and distills the second fraction and the first fraction; and
a third distillation separator (38) that receives supply of the first fraction subjected to the catalytic cracking treatment by the catalytic cracking unit (34) and distills the first fraction,
wherein the second distillation separator (36) separates at least one selected from the group consisting of a light hydrocarbon (C4-) having four or less carbon atoms, a naphtha fraction, a kerosene fraction, a gas oil fraction, or a fraction heavier than the gas oil from the second fraction subjected to the hydrogenation treatment and the first fraction subjected to the hydrocracking treatment, and
wherein the third distillation separator (38) separates at least one selected from the group consisting of a light hydrocarbon (C4-) having four or less carbon atoms, a gasoline fraction, a light cycle oil, or a clarified oil from the first fraction subjected to the catalytic cracking treatment.

### [Item 16]

The fuel production apparatus (1) according to Item 15,
wherein the second distillation separator (36) separates a fraction heavier than a gas oil, and
wherein the hydrocracking unit (32) receives supply of a portion or all the fraction heavier than the gas oil separated by the second distillation separator (36) and performs a hydrocracking treatment on the fraction.

### [Item 17]

The fuel production apparatus (1) according to Item 15 or 16,
wherein the third distillation separator (38) separates the clarified oil, and
wherein the catalytic cracking unit (34) receives supply of a portion or all of the clarified oil separated by the third distillation separator (38) and also performs the catalytic cracking treatment on the clarified oil.

### [Item 18]

A fuel production method comprising:
producing a synthesis gas containing carbon monoxide and hydrogen by using carbon dioxide and hydrogen;
producing a hydrocarbon (C5+) by using the synthesis gas;
separating at least a first fraction and a second fraction lighter than the first fraction from an effluent obtained from the producing a hydrocarbon;
performing a hydrocracking treatment on a portion of the first fraction; and
performing a catalytic cracking treatment on another portion of the first fraction.

### INDUSTRIAL APPLICABILITY

The present invention can be used in a fuel production apparatus and a fuel production method.

### REFERENCE SIGNS LIST

1 fuel production apparatus, 2 synthesis gas production unit, 4 hydrocarbon production unit, 6 first separator, 8 second separator, 14 reformer, 20 third separator, 22 fifth separator, 24 fourth separator, 28 first distillation separator, 30 hydrotreating unit, 32 hydrocracking unit, 34 catalytic cracking unit, 36 second distillation separator, 38 third distillation separator

## Claims

1. A fuel production apparatus comprising:
a synthesis gas production unit that produces a synthesis gas containing carbon monoxide and hydrogen by using carbon dioxide and hydrogen;
a hydrocarbon production unit that produces a hydrocarbon by using the synthesis gas;
a first distillation separator that separates at least a first fraction and a second fraction lighter than the first fraction from an effluent from the hydrocarbon production unit;
a hydrocracking unit that performs a hydrocracking treatment on a portion of the first fraction; and
a catalytic cracking unit that performs a catalytic cracking treatment on another portion of the first fraction.

2. The fuel production apparatus according to Claim 1, comprising:
a hydrotreating unit that performs a hydrogenation treatment on the second fraction.

3. The fuel production apparatus according to Claim 1 or 2, comprising:
a first separator that separates a recycle gas containing a light hydrocarbon having four or less carbon atoms from an effluent from the hydrocarbon production unit, and
performing at least one of supplying the recycle gas to the synthesis gas production unit so as to also use the recycle gas for producing a synthesis gas or supplying the recycle gas to the hydrocarbon production unit so as to also use the recycle gas for producing a hydrocarbon.

4. The fuel production apparatus according to Claim 3,
wherein the synthesis gas also contains carbon dioxide,
wherein the fuel production apparatus includes a second separator that separates carbon dioxide from the synthesis gas, and
wherein the synthesis gas production unit receives supply of the carbon dioxide separated by the second separator and also uses the carbon dioxide for producing the synthesis gas.

5. The fuel production apparatus according to Claim 3,
wherein the recycle gas also contains carbon monoxide,
wherein the fuel production apparatus includes a third separator that separates carbon monoxide from the recycle gas, and
wherein the hydrocarbon production unit receives supply of the carbon monoxide separated by the third separator and also uses the carbon monoxide for producing the hydrocarbon.

6. The fuel production apparatus according to Claim 3,
wherein the recycle gas also contains hydrogen,
wherein the fuel production apparatus includes a fourth separator that separates hydrogen from the recycle gas, and
wherein the hydrocarbon production unit receives supply of the hydrogen separated by the fourth separator and also uses the hydrogen for producing the hydrocarbon.

7. The fuel production apparatus according to Claim 4,
wherein the recycle gas also contains hydrogen, carbon monoxide, and carbon dioxide,
wherein the fuel production apparatus includes:
a third separator that separates carbon monoxide from the recycle gas; and
a fifth separator that receives supply of the recycle gas from which carbon monoxide has been separated by the third separator and separates hydrogen and carbon dioxide from the recycle gas,
wherein the second separator receives supply of the hydrogen and carbon dioxide separated by the fifth separator and separates the hydrogen and the carbon dioxide into hydrogen and carbon dioxide respectively, and
wherein the hydrocarbon production unit receives supply of the carbon monoxide separated by the third separator and the hydrogen separated by the second separator and also uses the carbon monoxide and the hydrogen for producing the hydrocarbon.

8. The fuel production apparatus according to Claim 3,
wherein the recycle gas also contains hydrogen and carbon monoxide,
wherein the fuel production apparatus includes:
a third separator that separates carbon monoxide from the recycle gas; and
a fourth separator that receives supply of the recycle gas from which carbon monoxide has been separated by the third separator and separates hydrogen from the recycle gas, and
wherein the hydrocarbon production unit receives supply of the carbon monoxide separated by the third separator and the hydrogen separated by the fourth separator and also uses the carbon monoxide and the hydrogen for producing the hydrocarbon.

9. The fuel production apparatus according to Claim 4,
wherein the recycle gas also contains hydrogen, carbon monoxide, and carbon dioxide,
wherein the fuel production apparatus includes:
a fifth separator that separates hydrogen and carbon dioxide from the recycle gas; and
a third separator that receives supply of the recycle gas from which hydrogen and carbon dioxide have been separated by the fifth separator and separates carbon monoxide from the recycle gas,
wherein the second separator receives supply of the hydrogen and carbon dioxide separated by the fifth separator and separates the hydrogen and the carbon dioxide into hydrogen and carbon dioxide, and
wherein the hydrocarbon production unit receives supply of the carbon monoxide separated by the third separator and the hydrogen separated by the second separator and also uses the carbon monoxide and the hydrogen for producing the hydrocarbon.

10. The fuel production apparatus according to Claim 3,
wherein the recycle gas also contains water and carbon monoxide,
wherein the fuel production apparatus includes:
a fourth separator that separates hydrogen from the recycle gas; and
a third separator that receives supply of the recycle gas from which hydrogen has been separated by the fourth separator and separates carbon monoxide from the recycle gas, and
wherein the hydrocarbon production unit receives supply of the hydrogen separated by the fourth separator and the carbon monoxide separated by the third separator and also uses the hydrogen and the carbon monoxide for producing the hydrocarbon.

11. The fuel production apparatus according to Claim 3, comprising:
a reformer that reforms the light hydrocarbon into methane,
wherein the synthesis gas production unit receives supply of the methane generated by the reformer and also uses the methane for producing the synthesis gas.

12. The fuel production apparatus according to Claim 11,
wherein the first distillation separator receives supply of an effluent from which the recycle gas has been separated by the first separator and separates the light hydrocarbon from the effluent, and
wherein the reformer receives supply of the light hydrocarbon from the first distillation separator and also reforms the light hydrocarbon into methane.

13. The fuel production apparatus according to Claim 11, comprising:
a hydrotreating unit that performs a hydrogenation treatment on the second fraction;
a second distillation separator that receives supply of the second fraction subjected to the hydrogenation treatment by the hydrotreating unit and the first fraction subjected to the hydrocracking treatment by the hydrocracking unit and distills the second fraction and the first fraction; and
a third distillation separator that receives supply of the first fraction subjected to the catalytic cracking treatment by the catalytic cracking unit and distills the first fraction,
wherein the second distillation separator separates the light hydrocarbon from the second fraction subjected to the hydrogenation treatment and the first fraction subjected to the hydrocracking treatment,
wherein the third distillation separator separates the light hydrocarbon from the first fraction subjected to the catalytic cracking treatment, and
wherein the reformer receives supply of the light hydrocarbon from at least one of the second distillation separator or the third distillation separator and also reforms the light hydrocarbon into methane.

14. The fuel production apparatus according to Claim 3,
wherein the recycle gas also contains carbon monoxide,
wherein the fuel production apparatus includes a third separator that separates carbon monoxide from the recycle gas, and
a reformer that receives supply of the recycle gas from which carbon monoxide has been separated by the third separator and reforms the light hydrocarbon contained in the recycle gas into methane,
wherein the hydrocarbon production unit receives supply of the carbon monoxide separated by the third separator and also uses the carbon monoxide for producing the hydrocarbon, and
wherein the synthesis gas production unit receives supply of the methane generated by the reformer and also uses the methane for producing the synthesis gas.

15. The fuel production apparatus according to Claim 2, comprising:
a second distillation separator that receives supply of the second fraction subjected to the hydrogenation treatment by the hydrotreating unit and the first fraction subjected to the hydrocracking treatment by the hydrocracking unit and distills the second fraction and the first fraction; and
a third distillation separator that receives supply of the first fraction subjected to the catalytic cracking treatment by the catalytic cracking unit and distills the first fraction,
wherein the second distillation separator separates at least one selected from the group consisting of a light hydrocarbon having four or less carbon atoms, a naphtha fraction, a kerosene fraction, a gas oil fraction, or a fraction heavier than the gas oil from the second fraction subjected to the hydrogenation treatment and the first fraction subjected to the hydrocracking treatment, and
wherein the third distillation separator separates at least one selected from the group consisting of a light hydrocarbon having four or less carbon atoms, a gasoline fraction, a light cycle oil, or a clarified oil from the first fraction subjected to the catalytic cracking treatment.

16. The fuel production apparatus according to Claim 15,
wherein the second distillation separator separates a fraction heavier than the gas oil, and
wherein the hydrocracking unit receives supply of a portion or all the fraction heavier than the gas oil separated by the second distillation separator and performs a hydrocracking treatment on the fraction.

17. The fuel production apparatus according to Claim 15,
wherein the third distillation separator separates the clarified oil, and
wherein the catalytic cracking unit receives supply of a portion or all of the clarified oil separated by the third distillation separator and also performs the catalytic cracking treatment on the clarified oil.

18. A fuel production method comprising:
producing a synthesis gas containing carbon monoxide and hydrogen by using carbon dioxide and hydrogen;
producing a hydrocarbon by using the synthesis gas;
separating at least a first fraction and a second fraction lighter than the first fraction from an effluent obtained from the producing a hydrocarbon;
performing a hydrocracking treatment on a portion of the first fraction; and
performing a catalytic cracking treatment on another portion of the first fraction.
